(19) Europäisches Patentamt

European Patent Office

Office européen des brevets

(11) **EP 1 045 250 A1**

## (12) EUROPEAN PATENT APPLICATION

(43) Date of publication:
**18.10.2000 Bulletin 2000/42**

(51) Int. Cl.[7]: **G01N 33/86**, C12Q 1/56

(21) Application number: **99830209.5**

(22) Date of filing: **12.04.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant:
**INSTRUMENTATION LABORATORY S.p.A.
I-20128 Milano (IT)**

(72) Inventor: **Preda, Luigi
20128 Milano (IT)**

(74) Representative:
**Long, Giorgio et al
Jacobacci & Perani
Via Senato, 8
20121 Milano (IT)**

(54) **A global test for evaluating the functionality of the thrombin/antithrombin system**

(57)     The present invention relates to an analytical test for evaluating the functionality of the thrombin/antithrombin system.

In particular, the present invention relates to an analytical method for evaluating the functionality of the thrombin/antithrombin system, comprising the following steps:

(a) mixing a sample of plasma to be analyzed with an agent promoting the inhibitory activity of antithrombin;
(b) adding a Factor II activating agent to the mixture produced in step (a);
(c) measuring the time ta taken to convert the fibrinogen of the mixture produced in step (b) into fibrin.

EP 1 045 250 A1

## Description

The present invention relates to an analytical test for evaluating the functionality of the thrombin/antithrombin system.

In brief, the heamostatic system may be seen as an equilibrium between a coagulant potential $P_c$ and an anticoagulant potential $P_{ant}$. A change in this equilibrium inevitably leads to pathological conditions and, in particular, a reduction in $P_{ant}$ may lead to thrombophilic conditions, whereas a reduction in $P_c$ creates a risk of haemorrhage.

The thrombin/antithrombin system plays an important role in coagulation biochemistry, particularly in quantitative terms. Thrombin (activated Factor II) in fact performs the following functions:

- converting fibrinogen (by detaching the A and B fibrinopeptides) into fibrin monomer which subsequently polymerizes to fibrin;
- activating Factor XIII, the function of which is to establish the bonds between fibrin polymer chains;
- directly activating Factor V and Factor VIII by means of a feedback mechanism, considerably increasing the $P_c$ of the system;
- activating protein C the activated form of which, together with protein cofactor S, is the basic enzyme of a natural anticoagulant system.

Thrombin activity is controlled fundamentally by the inhibitory action of antithrombin (AT) and, in part, of Heparin Cofactor II (HC II). It is known that the rate of thrombin inhibition by these natural anticoagulants is considerably increased in the presence of heparin and/or of glycosaminoglycanes (for example, dermatan sulphate).

The activation of Factor II with thrombin takes place physiologically by means of an intermediary known as meizothrombin (J. Rosing et al., Thromb Haemostas, 60(3), 355-360, 1988). This substance can also be produced "*in vitro*" by the action of the venom of some snakes. Meizothrombin also has enzymatic activity similar to that of thrombin, but the speed both of the reaction with fibrinogen and of the inhibition by AT differ considerably from those of thrombin.

Clearly, if an increase in the activity of the thrombin in circulation is not balanced by an adequate anticoagulant activity, it will lead to an increase in $P_c$ and hence to an increased thromboembolic risk.

Although global and specific tests for evaluating both the functionality of the protein C/protein S system and the plasma coagulant potential are already in use, these methods have been found inadequate for providing a biochemical explanation for a large number of thromboembolic episodes. In other words, it has been found that many patients with previous thrombosis showed absolutely normal parameters in the above-mentioned tests.

A proposed biochemical test recently reported in the literature (P.E. Makris et al., Blood Coagulation and Fibrinolysis 1998, 9: 665-666) is based on the addition of a complex of human antithrombin and heparin to a patient's plasma. The authors maintain that, by means of this test, they can identify patients with an anomalous resistance to the inhibition of Factor IIa, considering this to be a possible cause of thrombosis.

There is thus a great need to provide a new analytical method, alongside existing methods, which enables the possible appearance of thromboembolic episodes to be evaluated, if not in all cases, at least in a large percentage of cases.

This problem has been solved by the method of determining the functionality of the thrombin/antithrombin system as described and claimed in the appended claims.

The analytical method of the present invention provides for the following steps:

(a) mixing a sample of plasma to be analyzed with an agent promoting the inhibitory activity of antithrombin;
(b) adding a Factor II activating agent to the mixture produced in step (a);
(c) measuring the time $t_a$ taken to convert the fibrinogen of the mixture produced in step (b) into fibrin.
   The time taken ta measured depends both on the $P_c$ and on the $P_{ant}$ of the plasma under test.
   The method then continues with the following steps:
(d) mixing the same plasma sample as in point (a) with a suitable buffer which is free of the agent promoting the inhibitory activity of antithrombin;
(e) adding a Factor II activating agent to the mixture produced in point (d);
(f) measuring the time $t_0$ taken to convert the fibrinogen of the mixture produced in point (d) into fibrin.

The time $t_0$ measured depends solely on the $P_c$ of the plasma under test.

$\Delta_{sample} = t_a - t_0$ is then calculated.

It may be advantageous to normalize the $\Delta_{sample}$ value thus obtained by relating it to a $\Delta_{reference}$ obtained by subjecting a reference plasma (a mixture of the plasma of normal patients) to the method according to steps (a)-(f) and again calculating the reference difference $(t_a - t_0)_{reference}$. The normalized value is calculated as follows:

$$\Delta_N = \Delta_{sample}/\Delta_{reference}.$$

The agent promoting the inhibitory activity of antithrombin provided for in point (a) is selected from the group comprising non-fractionated heparin (molecular weight 1000-15000 Daltons) in non-salified form or as sodium, calcium or lithium salt, low molecular-weight heparin (LMW heparin with a molecular weight < 5000 Daltons) in non-salified form or as sodium, calcium or lithium salt, and dermatan sulphate.

The concentration of the promoter, dissolved in a suitable diluent such as, for example, water or suitably buffered water, is preferably between 4 and 8 U/ml, both for non-fractionated heparin and for LMW heparin, and between 15 and 25 $\mu$g/ml for dermatan sulphate.

A mixture of all of the components is preferably used. Non-fractionated heparin in fact performs its action in relation to the FIIa and FXa factors, whereas LWM heparin acts selectively solely on the FXa factor. The action of dermatan sulphate is directed exclusively towards the FIIa factor by means of both antithrombin and Heparin Cofactor II. The inhibition rate of HC II is greater than that of AT.

It has been found that the addition of LMW heparin alone, for example, would not show up functional anomalies relating to the FIIa factor or to Heparin Cofactor II.

The Factor II activating agent is preferably the venom of *Echis Carinatus* or a purified fraction thereof. The advantage of using this reagent is that the venom of *Echis Carinatus* activates FII directly and selectively.

The concentration of the Factor II activating agent, dissolved in a suitable solvent such as water, is preferably between 0.5 and 2 $\mu$g/ml.

The reagents used in point (a) and in point (b), and in point (e), respectively, are generally added to an aqueous solution containing $Ca^{2+}$ or $Mg^{2+}$ salts in a concentration of from 1 to 20 mmoles/l and $Na^+$ or $K^+$ salts in a concentration of from 50 to 200 mmoles/l, before being mixed with the plasma to be analyzed. This aqueous solution also constitutes the buffer used in step (d).

In a preferred embodiment of the method of the invention, the agent promoting the inhibition of antithrombin and the Factor II activating agent are mixed beforehand in the quantities indicated above and this mixture is then added to the plasma sample to be analyzed. In this variant of the method, steps (a) and (b) are thus combined in a single operative step. Similarly, steps (d) and (e) are combined in a single operative step. It has in fact been found that preliminary incubation of the plasma sample with heparin before the subsequent addition of the agent for activating the FII factor is not strictly necessary.

The reagents used in the present method are preferably used in lyophilized form.

The analytical method of the present invention is illustrated further by the following examples.

Example 1

100 $\mu$l of plasma, which was kept at 37°C throughout the process, was supplemented with 100 $\mu$l of an aqueous solution of 4.0 U/ml of sodic heparin, 5.0 mmoles/l of $Ca^{2+}$ salt and 150 mmoles/l of $Na^+$ salt. 100 $\mu$l of aqueous solution of *Echis Carinatus* venom with a concentration of 1.0 $\mu$g/ml was then added. The time $t_a$ necessary to coagulate the plasma was recorded.

100 $\mu$l of the same plasma, again at 37°C, was supplemented with 100 $\mu$l of an aqueous solution containing 5.0 mmoles/l of $Ca^{2+}$ salts and 150 mmoles/l of $Na^+$ salts and, subsequently, with 100 $\mu$l aqueous solution of *Echis Carinatus* venom with a concentration of 1.0 $\mu$g/ml. The time $t_0$ necessary to coagulate the plasma was recorded.

The difference

$$\Delta_{sample} = (t_a - t_0)_{sample}$$

was then calculated.

By repeating the steps given above on a reference plasma, a value

$$\Delta_{reference} = (t_a - t_0)_{reference}$$

was obtained.

The following parameters:

$$R = (t_0)_{sample}/(t_0)_{reference}$$

and

$$\Delta_N = \Delta_{sample}/\Delta_{reference}$$

were then calculated.

Example 2

100 μl of plasma, which was kept at 37°C throughout the process, was supplemented with 200 μl of an aqueous solution of 2.0 U/ml of sodic heparin, 0.5 μg/ml of *Echis Carinatus* venom, 2.5 mmoles/l of $Ca^{2+}$ salt and 75 mmoles/l of $Na^+$ salt. The time $t_a$ necessary to coagulate the plasma was recorded.
100 μl of the same plasma, again at 37°C, was supplemented with 200 μl of an aqueous solution containing 0.5 μg/ml of *Echis Carinatus* venom, 2.5 mmoles/l of $Ca^{2+}$ salts and 75 mmoles/l of $Na^+$ salts. The time $t_0$ necessary to coagulate the plasma was recorded.
The difference

$$\Delta_{sample} = (t_a - t_0)_{sample}$$

was then calculated.
By repeating the steps given above on a reference plasma, a value

$$\Delta_{reference} = (t_a - t_0)_{reference}$$

was obtained.
The following parameters:

$$R = (t_0)_{sample}/(t_0)_{reference}$$

and

$$\Delta_N = \Delta_{sample}/\Delta_{reference}$$

were then calculated.
The results obtained according to the methods of Examples 1 and 2 may be interpreted as follows:

Table 1

| result | significance | possible cause |
|---|---|---|
| R>1.25 | coagulating activity reduced | - Factor II activity reduced |
| | | - dis-fibrinogenaemia |
| R<0.90 | coagulating activity increased | - Factor II activity increased (physiological or 20210 GA mutation) |
| $\Delta_N$<0.75 | increased resistance to inhibition of FIIa | - reduction in AT activity (acquired or congenital) |
| | | - reduced HC II activity (acquired or congenital) |
| | | - increase in Factor II activity |
| | | - increase in fibrinogen content |
| | | - functional anomaly of Factor II |
| | | - functional anomaly of Factor X |

Table 2 gives the results obtained by analyzing the plasma of patients carrying some anomalies inherent in the thrombin/antithrombin system. In this case also, the methods of Examples 1 and 2 were followed.

Table 2

| Defect or anomaly | $t_0$ (sec) | $t_a$ (sec) | R | $\Delta_N$ |
|---|---|---|---|---|
| dis-fibrinogenaemia | 90 | 300 | 2.50 | 2.50 |
| fibrinogen (>700 mg/dl) | 45 | 130 | 0.80 | 0.60 |
| AT deficiency | 50 | 150 | 1.00 | 0.70 |
| HC II deficiency | 50 | 150 | 1.00 | 0.70 |
| Factor II (>120%) | | | | |
| physiological pregnancy | 45 | 140 | 0.80 | 0.60 |
| 20210 GA mutation | 45 | 140 | 0.80 | 0.60 |
| Factor II (<50%) | 90 | 300 | 2.50 | 2.50 |
| normal control | 50 | 190 | 1.00 | 1.00 |
| none known | 50 | 150 | 1.00 | 0.70 |
| none known | 45 | 150 | 0.80 | 0.70 |

It is thus clear from the foregoing and from Tables 1 and 2 that the analytical method of the present invention can provide a complete view of the functionality of the thrombin/antithrombin system.

The reagents used in the method of the present invention, particularly the agent promoting the inhibitory activity of AT and the FII factor activating agent, may be provided in a kit in which they may be present in separate or mixed form, in solution or in the solid state, preferably in lyophilized form.

**Claims**

1.   An analytical method of evaluating the functionality of the thrombin/antithrombin system, comprising the following steps:

   (a) mixing a sample of plasma to be analyzed with an agent promoting the inhibitory activity of antithrombin;
   (b) adding a Factor II activating agent to the mixture produced in step (a);
   (c) measuring the time $t_a$ taken to convert the fibrinogen of the mixture produced in step (b) into fibrin.

2.   A method according to Claim 1, comprising the following further steps:

   (d) mixing the same plasma sample as in point (a) with a suitable buffer which is free of the agent promoting the inhibitory activity of antithrombin;
   (e) adding a Factor II activating agent to the mixture produced in point (d);
   (f) measuring the time $t_0$ taken to convert the fibrinogen of the mixture produced in point (d) into fibrin.

3.   A method according to Claim 2, comprising the following further steps:

   (g) calculating $\Delta_{sample} = t_a - t_0$ ;
   (h) repeating the sequence of steps (a) to (g) on a reference plasma;
   (i)   calculating   the   normalized   value   $\Delta_N = \Delta_{sample}/\Delta_{reference}$   and   calculating   the   ratio $R = (t_0)_{sample}/(t_0)_{reference}$ .

4.   A method according to any one of Claims 1 to 3, in which the agent promoting the inhibitory activity of antithrombin is selected from the group which comprises non-fractionated heparin in non-salified form or as sodium, calcium or lithium salt, LWM heparin in non-salified form or as sodium, calcium or lithium salt, and dermatan sulphate, or preferably a mixture thereof.

5.   A method according to Claim 4, in which the non-fractionated heparin and/or the LMW heparin have a concentration in aqueous solution of between 4 and 8 U/ml and the dermatan sulphate has a concentration of between 15 and 25 µg/ml.

6. A method according to any one of Claims 1 to 5, in which the Factor II activating agent is *Echis Carinatus* venom or a purified fraction thereof, preferably in aqueous solution with a concentration of between 0.5 and 2 µg/ml.

7. A method according to any one of Claims 1 to 6, in which the reagent used in step (a) is placed beforehand in an aqueous solution containing $Ca^{2+}$ or $Mg^{2+}$ salts in a concentration of from 1 to 20 mmoles/l and $Na^+$ or $K^+$ salts in a concentration of from 50 to 200 mmoles/l.

8. A method according to any one of Claims 1 to 7, in which the agent promoting the inhibitory activity of antithrombin and the Factor II activating agent are mixed beforehand in solution or in solid form.

9. A method according to Claim 8, in which the solid form is a lyophilized form.

10. A method according to any one of Claims 1 to 9, in which the buffer used in step (d) is an aqueous solution containing $Ca^{2+}$ or $Mg^{2+}$ salts in a concentration of from 1 to 20 mmoles/l and $Na^+$ or $K^+$ salts in a concentration of from 50 to 200 mmoles/l.

11. A kit for determining the functionality of the thrombin/antithrombin system, comprising an agent promoting the inhibitory activity of antithrombin and a Factor II activating agent in separate form or as a mixture, in solution or in solid form.

12. A kit according to Claim 11, in which the agent promoting the inhibitory activity of antithrombin is selected from the group comprising non-fractionated heparin in non-salified form or as sodium, calcium or lithium salt, LWM heparin in non-salified form or as sodium, calcium or lithium salt and dermatan sulphate, or preferably a mixture thereof.

13. A kit according to Claim 11 or Claim 12, in which the Factor II activating agent is *Echis Carinatus* venom or a purified fraction thereof.

14. A composition comprising a mixture of non-fractionated heparin in non-salified form or as sodium, calcium or lithium salt, LWM heparin in non-salified form or as sodium, calcium or lithium salt, and/or dermatan sulphate and *Echis Carinatus* venom or a purified fraction thereof, in lyophilized form.

## EUROPEAN SEARCH REPORT

European Patent Office

**Application Number**

EP 99 83 0209

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 780 255 A (PREDA LUIGI) 14 July 1998 (1998-07-14) * abstract * | 1,11 | G01N33/86 C12Q1/56 |
| X | DATABASE MEDLINE 'Online! US NATIONAL LIBRARY OF MEDICINE (NLM), BETHESDA, MD, US NOWAK G ET AL: "Quantitative determination of hirudin in blood and body fluids." retrieved from STN Database accession no. 96401341 XP002116840 * abstract * & SEMINARS IN THROMBOSIS AND HEMOSTASIS, (1996) 22 (2) 197-202. , | 1 | |
| Y | EP 0 814 155 A (EISAI CO LTD) 29 December 1997 (1997-12-29) * examples 1,2 * | 14 | |
| Y | GB 1 157 593 A (S.E.M.S.) 9 July 1969 (1969-07-09) * page 1, line 17 – line 28 * | 14 | **TECHNICAL FIELDS SEARCHED (Int.Cl.7)** G01N C12Q |
| A | WO 92 07954 A (BAXTER DIAGNOSTICS INC) 14 May 1992 (1992-05-14) * page 3, line 9 – page 4, line 34 * | 1,11 | |
| A | US 5 716 795 A (MATSCHINER JOHN T) 10 February 1998 (1998-02-10) * the whole document * | 1 | |
| A | WO 93 07491 A (UNIV NEBRASKA) 15 April 1993 (1993-04-15) * examples * | 1 | |

-/--

The present search report has been drawn up for all claims

| Place of search | Date of completion of the search | Examiner |
|---|---|---|
| THE HAGUE | 29 September 1999 | Moreno, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

### EUROPEAN SEARCH REPORT

**European Patent Office**

**Application Number**

EP 99 83 0209

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 4 106 990 A (KARGES HERMANN ERICH ET AL) 15 August 1978 (1978-08-15) * the whole document * | 1 | |
| | | | **TECHNICAL FIELDS SEARCHED (Int.Cl.7)** |

The present search report has been drawn up for all claims

| Place of search | Date of completion of the search | Examiner |
|---|---|---|
| THE HAGUE | 29 September 1999 | Moreno, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 99 83 0209

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-1999

| Patent document cited in search report | | Publication date | Patent family member(s) | | Publication date |
|---|---|---|---|---|---|
| US 5780255 | A | 14-07-1998 | CA | 2224465 A | 27-12-1996 |
| | | | EP | 0830608 A | 25-03-1998 |
| | | | JP | 11504718 T | 27-04-1999 |
| | | | WO | 9642018 A | 27-12-1996 |
| EP 0814155 | A | 29-12-1997 | WO | 9627660 A | 12-09-1996 |
| GB 1157593 | A | 09-07-1969 | BE | 709370 A | 15-07-1968 |
| | | | DE | 1667914 A | 16-06-1971 |
| | | | FR | 6256 M | 19-08-1968 |
| | | | US | 3869548 A | 04-03-1975 |
| WO 9207954 | A | 14-05-1992 | AT | 121139 T | 15-04-1995 |
| | | | AU | 650941 B | 07-07-1994 |
| | | | AU | 1015492 A | 25-06-1992 |
| | | | CA | 2070813 A,C | 06-05-1992 |
| | | | DE | 69108896 D | 18-05-1995 |
| | | | DE | 69108896 T | 24-08-1995 |
| | | | DK | 509086 T | 03-07-1995 |
| | | | EP | 0509086 A | 21-10-1992 |
| | | | ES | 2073908 T | 16-08-1995 |
| | | | JP | 5503223 T | 03-06-1993 |
| | | | US | 5702912 A | 30-12-1997 |
| US 5716795 | A | 10-02-1998 | US | 5525478 A | 11-06-1996 |
| | | | AU | 2870992 A | 03-05-1993 |
| | | | WO | 9307491 A | 15-04-1996 |
| WO 9307491 | A | 15-04-1993 | AU | 2870992 A | 03-05-1993 |
| | | | US | 5525478 A | 11-06-1996 |
| | | | US | 5716795 A | 10-02-1998 |
| US 4106990 | A | 15-08-1978 | DE | 2601372 A | 28-07-1977 |
| | | | AT | 350193 B | 10-05-1979 |
| | | | AU | 515025 B | 12-03-1981 |
| | | | AU | 2129277 A | 20-07-1978 |
| | | | BE | 850453 A | 18-07-1977 |
| | | | CA | 1079166 A | 10-06-1980 |
| | | | CH | 626919 A | 15-12-1981 |
| | | | DK | 15077 A | 16-07-1977 |
| | | | ES | 455030 A | 01-04-1978 |
| | | | FR | 2338496 A | 12-08-1977 |
| | | | GB | 1569392 A | 11-06-1980 |
| | | | JP | 1312885 C | 28-04-1986 |
| | | | JP | 52088094 A | 22-07-1977 |
| | | | JP | 60033480 B | 02-08-1985 |

For more details about this annex : see Official Journal of the European Patent Office, No. 12/82

9

**ANNEX TO THE EUROPEAN SEARCH REPORT
ON EUROPEAN PATENT APPLICATION NO.**

EP 99 83 0209

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-1999

| Patent document cited in search report | Publication date | Patent family member(s) | Publication date |
|---|---|---|---|
| US 4106990 A | | ZA 7700210 A | 28-12-1977 |

EPO FORM P0459

For more details about this annex : see Official Journal of the European Patent Office, No. 12/82